# EUROPEAN PATENT SPECIFICATION

(11) **EP 3 937 914 B1**
(45) Date of publication and mention of the grant of the patent: **09.08.2023**
(21) Application number: 20712673.1
(22) Date of filing: 13.03.2020
(51) Int. Cl.: A61K 31/05, A61K 31/08, A61K 31/192, A61K 31/352, A61K 31/485, A61K 45/06, A61P 35/00, A61K 31/555, A61K 31/7068

(54) **CANCER TREATMENT COMPRISING NALTREXONE AND A CANNABINOID**
KREBSBEHANDLUNG MIT NALTREXON UND EINEM CANNABINOID
TRAITEMENT CONTRE LE CANCER COMPRENANT DE LA NALTREXONE ET UN CANNABINOÏDE

(30) Priority: 15.03.2019 GB 201903546
(43) Date of publication of application: 19.01.2022
(73) Proprietor: LDN Pharma Limited, London EC2V 7BG (GB)
(72) Inventor: LIU, Wai, London EC2V 7BG (GB); DALGLEISH, Angus, London EC2V 7BG (GB)
(74) Representative: Gill Jennings & Every LLP
(86) International application number: PCT/GB2020/050638
(87) International publication number: WO 2020/188255

(56) References cited:
- EP-A1- 3 482 751
- WO-A1-2014/181131
- WO-A1-2015/189597
- WO-A1-2019/193112
- GB-A- 2 460 672

## Description

### Field of the invention

The invention relates to regimes of drug administration and drug combinations for use in the treatment of cancer.

### Background to the invention

Cannabinoids are a class of phenolic compounds abundantly produced in plants of the *Cannabis* genus. Various cannabinoid compounds have long been known to exhibit psychotropic effects in humans and are widely used recreationally, despite such use being illegal in many jurisdictions.

However, recent research has shown that certain cannabinoids are of therapeutic value against a diverse array of pathologies such as inflammatory disorders, neurodegenerative and psychiatric disorders, chronic pain, anxiety and PTSD. While cannabinoids are currently used to combat the wasting, emesis and nausea associated with cancer treatment, evidence exists which suggests that certain cannabinoid compounds may be effective in treating the underlying pathologies of cancers. Presently available evidence suggests that they do this through disruption of cancer cell migration, adhesion, and tumour vascularisation.

An endogenous cannabinoid-mediated signalling system functions in mammals, and on the balance of evidence probably in most other vertebrates too. In humans, two cannabinoid receptors have been identified and named CB1 and CB2, both of which are part of the G protein-coupled receptor superfamily.

Pharmacological evidence to date suggests that activation of CB1 is responsible for the majority of the psychotropic effects of cannabinoid intake, while therapeutic effects are mostly mediated by CB2. As such, ideal therapeutic molecules will activate CB2 in preference to CB1, alleviating unwanted psychotropic side effects. Cannabidiol (CBD), a component of *Cannabis* extract, is a cannabinoid with this binding profile which has recently generated considerable interest, but whose full mechanism of action is still being elucidated.

In general however, natural cannabinoids do not tend to show high specificity for one or other receptor, but newly developed synthetic cannabinoids are available which bind more specifically, for example JWH-133 and SR141716 (Cridge & Rosengren 2013). These compounds have been shown to inhibit tumour growth and cancer cell viability both *in vitro* and in various tumour-implanted mouse models.

It is sometimes the case that receptors in one signalling pathway, responsive to a particular ligand, can have their expression levels or downstream effects altered by a change in the signalling output of another seemingly separate signalling pathway. Such cross-modulation has been demonstrated between, for example, the signalling pathways following activation of the Growth Hormone and insulin receptors.

### Summary of the invention

The invention is defined in the appended set of claims. It has been found by the present inventors that the inhibition of the proliferation of cancer cells by treatment with a cannabinoid and a chemotherapeutic agent is brought about more effectively by combined treatment with low dose naltrexone (LDN) or 6-β-naltrexol (6BN), a metabolite of naltrexone. They also found that the effectiveness of such treatment is surprisingly dependent on the order in which the LDN or 6BN and the cannabinoid are administered, with the most effective regime being a phase of treatment with LDN or 6BN followed by a phase of treatment with a cannabinoid.

The inventors have also found that treatment with LDN or 6BN brings about a significant increase in levels of the CB2 receptor in cancer cells, thus allowing the cannabinoid to be more effective.

According to a first aspect of the invention, there is provided a pharmaceutical composition comprising naltrexone, or a metabolite thereof selected from the group consisting of 6-β-naltrexol, 2-hydroxy-3-methoxy-6β-naltrexol and 2-hydroxy-3-methoxy-naltrexone, or an analogue selected from the group consisting of methylnaltrexone, naloxone, nalmefene and nalorphine, for use in the treatment of cancer within a subject, wherein the subject is to be administered a chemotherapeutic agent before, during or following the treatment and wherein a therapeutically effective amount of the naltrexone or metabolite thereof or analogue is to be administered to the subject in a first treatment phase, and wherein after the first treatment phase the subject is to be administered a therapeutically effective amount of a cannabinoid in a second treatment phase.

According to a second aspect of the invention, there is provided a pharmaceutical composition comprising a cannabinoid for use in the treatment of cancer within a subject, wherein said subject is characterised in having undergone a first treatment phase during which the subject has been administered a therapeutically effective amount of naltrexone, or a metabolite thereof selected from the group consisting of 6-β-naltrexol, 2-hydroxy-3-methoxy-6β-naltrexol and 2-hydroxy-3-methoxy-naltrexone, or an analogue selected from the group consisting of methylnaltrexone, naloxone, nalmefene and nalorphine, and wherein following the first treatment phase a therapeutically effective amount of said cannabinoid is to be administered to the subject, and wherein the subject is to be administered a chemotherapeutic agent before, during or following the treatment.

According to a third aspect of the invention, there is provided a preparation comprising naltrexone, or a metabolite thereof selected from the group consisting of 6-β-naltrexol, 2-hydroxy-3-methoxy-6β-naltrexol and 2-hydroxy-3-methoxy-naltrexone, or an analogue selected from the group consisting of methylnaltrexone, naloxone, nalmefene and nalorphine, and a cannabinoid, and a chemotherapeutic agent for use in the treatment of cancer within a subject, wherein the naltrexone or metabolite thereof or analogue is provided in a therapeutically effective amount to be administered in a first treatment phase, wherein the cannabinoid is provided in a therapeutically effective amount to be administered in a second treatment phase following the first treatment phase and wherein the chemotherapeutic agent is to be provided in a therapeutically effective amount before, during or following the treatment.

According to a fourth aspect of the invention, there is provided a method for determining the suitability of a subject with cancer for treatment with a cannabinoid in a second treatment phase, said subject characterised in having undergone a first treatment phase as defined above, the method comprising the steps of:
i. contacting a sample obtained from the subject after or during a recovery phase with a probe specific for CB2;
ii. determining the concentration of CB2 within the sample; and
iii. comparing the concentration of CB2 within the sample with a concentration of CB2 determined from a sample obtained from the subject before the first treatment phase,
wherein if the CB2 concentration has increased by at least two-fold after the first treatment phase, the subject is suitable for the second treatment phase, and wherein the subject is or has been administered a chemotherapeutic agent.

### Brief Description of the Figures

The invention is described with reference to the accompanying drawings, wherein:
**Figure 1** shows the effects of treatment for two days with naltrexone at 10 nM (LDN), 6-β-naltrexol at 1µM (6-1), or 6-β-naltrexol at 10 µM (6-2) on cultures of the MCF7 breast cancer cell line. Levels of Bcl2-associated death promoter (BAD), p21 protein, the opioid receptors kappa 1 (OPRK1) and mu 1 (OPRM1), and the cannabinoid receptors CBR1 (CB1) and CBR2 (CB2) are visualised by Western blot (left) against GAPDH as a loading control, and quantified via density analysis relative to GAPDH (right).
**Figure 2** shows the effect of various two-stage treatments on the proliferation and viability of MCF7 cells. Each treatment lasted four days and consisted of two days of treatment with the first agent (UN=control, LDN=Low Dose Naltrexone at 10 nM CBD=Cannabidiol, 6BN=6-β-naltrexol) before the second-named agent was introduced for a further two days.
**Figure 3** shows the effect of various two-stage treatments on the proliferation (3A) and viability (3B) of A549 cells in accordance with Example 3 (0 = nothing administered; LC = LDN and CBD; G = gemcitabine; P = oxaliplatin).
**Figure 4** shows the effect of various two-stage treatments on the proliferation (4A) and viability (4B) of HCT116 cells in accordance with Example 3 (0 = nothing administered; LC = LDN and CBD; G = gemcitabine; P = oxaliplatin).

### Detailed Description of the invention

The invention provides a specific therapeutic regimen for treating cancer in a subject, wherein a cannabinoid is administered after administration of Low Dose Naltrexone (LDN), a metabolite of naltrexone selected from the group consisting of 6-β-naltrexol, 2-hydroxy-3-methoxy-6β-naltrexol and 2-hydroxy-3-methoxy-naltrexone, or an analogue thereof selected from the group consisting of methylnaltrexone, naloxone, nalmefene and nalorphine, and wherein the subject is administered a chemotherapeutic agent before, during or following the therapeutic regimen.

The phased administration of LDN and then a cannabinoid has been found by the inventors to have a priming effect on the body's immune cells and the cancer cells, which in combination with a chemotherapeutic agent consequently inhibits cancer cell proliferation more effectively than separate or simultaneous administration, or phased administration of a cannabinoid and then LDN, or continuous administration of only a chemotherapeutic agent.

It was also found that levels of the CB2 receptor increased significantly in MCF7 cells after treatment with LDN. Without wishing to be bound by theory, this increase in CB2, which is the therapeutic target of cannabinoids such as CBD, is feasibly a contributing factor to the increase in the effectiveness of the treatment with CBD.

The skilled person will be able to carry out the phased administration of the therapeutic agents as described. The invention can be further understood with reference to the following definitions:
As used herein, "naltrexone" refers to the compound 17-cyclopropylmethyl-4.5α-epoxy-3,14-dihydroxymorphinan-6-one (with IUPAC name (4R,4aS,7aR,12bS)-3-(cyclopropylmethyl)-4a,9-dihydroxy-2,4,5,6,7a,13-hexahydro-1H-4,12-methanobenzofuro[3,2-e]isoquinoline-7-one) and pharmaceutically acceptable salts, solvates, hydrates, racemates, stereoisomers, clathrates, polymorphs and prodrugs thereof. Analogues thereof are also envisaged for use as per the invention. Suitable analogues include methylnaltrexone, naloxone, nalmefene and nalorphine.

The naltrexone is typically in its hydrochloride salt form.

"Low Dose Naltrexone" (LDN) refers to naltrexone administered in "low" doses of less than 0.5 mg/kg, preferably less than 0.2 mg/kg, more preferably between 0.01 mg/kg and 0.08 mg/kg, even more preferably between 0.03 mg/kg and 0.06 mg/kg, most preferably between 0.04 mg/kg and 0.05 mg/kg. Typically, a low dose is up to 3mg per day in total, per patient.

The metabolites of naltrexone of this invention are 6-β-naltrexol, 2-hydroxy-3-methoxy-6β-naltrexol and 2-hydroxy-3 methoxy-naltrexone.

The preferred metabolite of naltrexone is 6-β-naltrexol (6BN), which as used herein refers to the compound N-cyclopropylmethyl-7,8-dihydro-14-hydroxynorisomorphine (with IUPAC name (4R,4aS,7R,7aR,12bS)-3-(cyclopropylmethyl)-1 ,2,4,5,6,7,7a, 13-octahydro-4,12-methanobenzofuro[3,2-e]isoquinoline-4a,7,9-triol), and pharmaceutically acceptable salts, solvates, hydrates racemates, stereoisomers, clathrates, polymorphs, and prodrugs thereof.

The 6BN or analogues etc, may also be administered at "low dose". In this context, a "low dose" can be the same as that outlined above for naltrexone.

Cannabinoids are a class of compounds understood by the skilled person which comprise those abundantly made by plants of the *Cannabis* genus, as well as endocannabinoids which are synthesised in animals. Synthetic compounds active against CB receptors are also envisaged. When used herein the term can refer to any cannabinoid, but is preferably selected from the list containing cannabidiol, cannabidiolic acid, cannabinol, cannabigerol, cannabivarin, tetrahydrocannabivarin, cannabidivarin, cannabichromene, arachidonoylethanolamine, 2-arachidonoylglycerol, 2-arachidonoyl glyceryl ether, N-arachidonoyl dopamine, virodhamine, dronabinol, nabilone, rimonabant, R-(+)-Met-anandamide, WIN-55,212-2, HU-210, JWH-133, SR141716, SR144528 or combinations thereof, or pharmaceutically acceptable salts, solvates, hydrates, racemates, stereoisomers, clathrates, polymorphs, prodrugs, and analogues thereof which bring about equivalent effects.

As used herein "chemotherapy" and "chemotherapeutic" have their conventional meaning in the art. The term "anti-cancer agent" is used synonymously with "chemotherapeutic agent". For the avoidance of doubt, in this instance a cannabinoid is not an anti-cancer agent.

In certain embodiments, the chemotherapy involves administering an anti-cancer agent selected from the group consisting of PI3-kinase inhibitors, AKT inhibitors, taxanes, antimetabolites, alkylating agents, cell cycle inhibitors, topoisomerase inhibitors and cytotoxic antibodies.

Where the chemotherapeutic agent is a PI3-kinase inhibitor, suitable examples include, but are not limited to, wortmannin, LY294002, demethoxyviridin, IC87114, NVP-BEZ235, BAY 80-6946, BKM120, GDC-0941, GDC-9080; including combinations thereof; and pharmaceutically acceptable salts, solvates, hydrates, stereoisomers, clathrates and prodrugs of any of the above.

Where the anti-cancer agent is an AKT inhibitor, suitable examples include, but are not limited to, MK-2206, GSK690693, perifosine, PHT-427, AT7867, honokiol, PF-04691502; including combinations thereof; and pharmaceutically acceptable salts, solvates, hydrates, stereoisomers, clathrates and prodrugs of any of the above.

Where the anti-cancer agent is a taxane, suitable examples include, but are not limited to, paclitaxel and docetaxel; including combinations thereof; and pharmaceutically acceptable salts, solvates, hydrates, stereoisomers, clathrates and prodrugs of any of the above.

Where the anti-cancer agent is an antimetabolite, suitable examples include, but are not limited to, methotrexate, 5-fluorouracil, capecitabin, cytosinarabinoside (Cytarabin), gemcitabine, 6-thioguanin, pentostatin, azathioprin, 6-mercaptopurin, fludarabin and cladribin; including combinations thereof; and pharmaceutically acceptable salts, solvates, hydrates, stereoisomers, clathrates and prodrugs of any of the above.

Where the anti-cancer agent is an alkylating agent, suitable examples include, but are not limited to, mechlorethamine, cyclophosphamide, ifosfamide, trofosfamide, melphalan (L-sarcolysin), chlorambucil, hexamethylmelamine, thiotepa, busulfan, carmustine (BCNU), streptozocin (streptozotocin), dacarbazine (DTIC; dimethyltriazenoimidazol ecarboxamide) temozolomide and oxaliplatin; including combinations thereof; and pharmaceutically acceptable salts, solvates, hydrates, stereoisomers, clathrates and prodrugs of any of the above.

Where the anti-cancer agent is a cell cycle inhibitor, suitable examples include, but are not limited to, Epothilone, Vincristine, Vinblastine, UCN-01, 17AAG, XL844, CHIR-124, PF-00477736, CEP-3891, Flavopiridol, berberine, P276-00, terameprocol, isoflavone daidzein, BI2536, BI6727, GSK461364, Cyclapolin, ON-01910, NMS-P937, TAK-960, Ispinesib, Monastrol, AZD4877, LY2523355, ARRY-520, MK-0731, SB743921, GSK923295, Lonafarnib, proTAME, Bortezomib, MLN9708, ONX0912, CEP-18770; including combinations thereof; and pharmaceutically acceptable salts, solvates, hydrates, stereoisomers, clathrates and prodrugs of any of the above; particularly suitable examples of cell cycle inhibitors include, but are not limited to, Hespaeradin, ZM447439, VX-680, MLN-8054, PHA-739358, AT-9283, AZD1152, MLN8237, ENMD2076, SU6668; including combinations thereof; and other inhibitors of Aurora kinases; and pharmaceutically acceptable salts, solvates, hydrates, stereoisomers, clathrates and prodrugs of any of the above.

The anti-cancer agent may be a checkpoint inhibitor. Checkpoint inhibitors are a form of cancer immunotherapy with currently approved inhibitors that target the molecules CTLA4, PD-1, and PD-L1.

"Simultaneous" administration, as defined herein, includes the administration of substances within about 2 hours or about 1 hour or less of each other, even more preferably at the same time.

"Separate" administration, as defined herein, includes the administration of substances, more than about 12 hours, or about 8 hours, or about 6 hours or about 4 hours or about 2 hours apart.

"Sequential" administration, as defined herein, includes the administration of substances each in multiple aliquots and/or doses and/or on separate occasions.

The anti-cancer agent may be administered to the subject before, during or following the treatment regimen. During the treatment regimen the anti-cancer agent may be administered to the subject at any time, such as during the first treatment phase or during the second treatment phase.

During the first treatment phase, the anti-cancer agent may be administered to the patient before and/or after administration of the naltrexone or a metabolite thereof or an analogue thereof. The anti-cancer agent and the naltrexone or a metabolite thereof or an analogue thereof may be administered simultaneously, sequentially or separately, preferably simultaneously. The anti-cancer agent may also be continued to be administered to the patient after treatment with the naltrexone ceases, so the anti-cancer agent is administered in the first and second treatment phases.

During the second treatment phase, the anti-cancer agent may be administered to the patient and before and/or after administration of the cannabinoid. The anti-cancer agent and the cannabinoid may be administered simultaneously, sequentially or separately, preferably simultaneously. The anti-cancer agent may also be continued to be administered to the patient after treatment with the cannabinoid ceases, so the anti-cancer agent is also administered following the treatment regimen.

In another embodiment, the therapy may also include administering Vitamin D to the subject. The Vitamin D may be administered to the subject before, during or following the treatment regimen. During the treatment regimen the Vitamin D may be administered to the subject at any time, such as during the first treatment phase or during the second treatment phase. Preferably, the administration of Vitamin D in the patient should occur before the disclosed treatment regimen begins.

During the first treatment phase, the Vitamin D may be administered to the patient before and/or after administration of the naltrexone or a metabolite thereof or an analogue thereof. The Vitamin D and the naltrexone or a metabolite thereof or an analogue thereof may be administered simultaneously, sequentially or separately, preferably simultaneously. The Vitamin D may also be continued to be administered to the patient after treatment with the naltrexone ceases, so the Vitamin D is administered in the first and second treatment phases.

During the second treatment phase, the Vitamin D may be administered to the patient before and/or after administration of the cannabinoid. The Vitamin D and the cannabinoid may be administered simultaneously, sequentially or separately, preferably simultaneously. The Vitamin D may also be continued to be administered to the patient after treatment with the cannabinoid ceases, so the Vitamin D is also administered following the treatment regimen.

Preferably during the treatment regimen the Vitamin D should be administered to the subject on a daily basis to maintain the correct Vitamin D levels throughout all phases of treatment.

As used herein, "vitamin D" refers to vitamin D and any intermediate or product of a metabolic pathway of vitamin D that result in a metabolite that is capable of boosting the cytostatic effect of the active. Metabolite may refer to a vitamin D precursor, which can be incorporated into a vitamin D synthetic pathway occurring naturally within the subject to undergo the therapy of the invention. Alternatively, metabolite may refer to a molecule derived from an anabolic or catabolic process that utilizes vitamin D. Non-limiting examples of vitamin D metabolites include ergocalciferol, cholecalciferol, calcidiol, and calcitriol, 1a-hydroxycholecalciferol, 25-hydroxycholecalciferol, 1a,25-hydroxycholecalciferol, 24,25-hydroxycholecalciferol. An "active" metabolite is a metabolite that can be used in the context of the present invention. Dosage regimes of vitamin D or active metabolites thereof will be well known to the person skilled in the art. The term vitamin D also encompasses pharmaceutically acceptable salts of any of the above. A particularly suitable metabolite of vitamin D for use in the present invention is calcitriol.

In certain embodiments, where the agent is 6-β-naltrexol, 6-β-naltrexol is to be administered in an amount effective to increase the blood plasma concentration of 6-β-naltrexol to at least 0.34 ng/ml, preferably at least 3.4 ng/ml, more preferably at least 34 ng/ml, or most preferably at least 340 ng/ml. In certain embodiments, 6-β-naltrexol is to be administered in an amount effective to increase the blood plasma concentration of 6-β-naltrexol to within the range of 0.3 ng/ml to 3,400 ng/ml, preferably to within the range of from 34 ng/ml to 3,400 ng/ml, more preferably within the range of 340 ng/ml to 3,400 ng/ml. The amount effective to achieve such an amount can be determined using any number of conventional techniques known to the person skilled in the art. For example, the skilled person could perform mass spectrometry on a blood plasma sample obtained from the subject in order to determine the increase in the concentration of 6-β-naltrexol within the sample after administration of an amount of 6-β-naltrexol. The effective amount is the amount determined to bring about the desired increase in blood plasma concentration. Typically, the naltrexol will be administered in an amount up to 3mg per day per patient.

The cannabinoid can be administered at conventional amounts based on the particular cannabinoid and the details of the patient. In certain embodiments, the cannabinoid is to be administered each day in doses of between 10 mg and 1000 mg, preferably between 200 and 800 mg, more preferably between 300 and 500 mg.

The chemotherapeutic agent can be administered at conventional amounts based on the particular chemotherapeutic agent and the details of the patient. In certain embodiments, the chemotherapeutic agent is to be administered each day in doses as used in standard practice.

In certain embodiments, the vitamin D product is to be administered to the patient in an amount sufficient to bring the subject's blood vitamin D concentration to at least 40 ng/ml, more preferable at least 50 ng/ml. Preferably, the blood vitamin D concentration is raised to within a range of from 40 to 220 ng/ml, more preferably the blood vitamin D concentration is raised to within a range of from 40 to 90 ng/ml.

A sufficient amount can be determined by the skilled person by making a routine assessment of certain parameters of the patient to undergo the administration, such as, but not limited to, age, weight, gender, history of illness and/or other lifestyle factors including smoking, alcohol consumption and the level of exercise. Furthermore the skilled person can ascertain whether a dose has been sufficient to raise the vitamin D blood concentration to a sufficient amount by performing routine biochemical and analytical assays on a biological sample obtained from the subject. Preferably, the sample upon which said analysis is to be performed is blood. Examples of such well known assays include but are not limited to mass spectrometry, where the level of vitamin D or active metabolites thereof can be quantitatively measured. A sufficient amount is therefore an amount that achieves the desired blood vitamin D concentration. The desired concentration can be achieved after single administration or after repeated administrations of a dose of vitamin D or an active metabolite thereof. Where the vitamin D product and the naltrexone product are to be administered simultaneously, it is immaterial whether the vitamin D blood concentration is within the desired range prior to administration of the naltrexone product, provided that the vitamin D product is administered in an amount sufficient to raise the blood vitamin D concentration to within the desired concentration range. Other methods for determining the concentration of vitamin D or active metabolites thereof within a biological sample obtained from the patient will be well known to the skilled person. In certain embodiments, the amount of the vitamin D sufficient to raise the blood vitamin D concentration to beyond a certain level is referred to as the "therapeutically effective amount" of the vitamin D product.

As used herein, a "preparation" can refer to a substance or a collection of substances, in the form of one or more compositions intended for use either simultaneously or non-simultaneously.

The method of administration is not particularly limited for any therapeutic agent, but in various embodiments of the invention, LDN, cannabinoids and chemotherapeutic agents are administered via the oral, buccal, sublingual, nasal, pulmonary, intravenous, rectal, topical, and transdermal routes. For LDN administration is preferably oral, for cannabinoids it is preferably sublingual and for chemotherapeutic agents it is preferably oral or intravenous.

The treatment regimen envisaged comprises a "first treatment phase" and a "second treatment phase". In the first treatment phase, a therapeutically effective amount of LDN, a metabolite thereof, or an analogue of either is administered. In the second treatment phase, an effective amount of one or more cannabinoids is administered. The chemotherapeutic agent may be administered before, during and/or following the treatment regimen.

In one preferred embodiment, the chemotherapeutic agent is administered following the treatment regimen. This can be advantageous as it allows the immune cells and the cancer cells to be "primed" (activated) and thus more effective at reducing the cancer when the chemotherapeutic agent is then administered.

It is preferred that the second treatment phase begins only after 1 to 7 days have elapsed from the start of the first treatment phase, more preferably 1 to 4 days, most preferably 1 to 2 days, a "day" being a continuous period of 24 hours.

In a further preferred embodiment of the invention, there is a "recovery phase" in between the end of the first treatment phase and the start of the second treatment phase. During the recovery phase no LDN, cannabinoid or chemotherapeutic agent is administered. In one embodiment the recovery phase has a duration of at least two days, preferably no more than 1 week apart, most preferably the duration is two days.

As used herein, the terms "treating" and "treatment" and "to treat" refer to both therapeutic measures that cure, slow down, and/or halt progression of a diagnosed pathologic condition or disorder, and also to prophylactic or preventative measures that prevent and/or slow the development of a targeted pathologic condition or disorder. Therefore, those in need of treatment include those already with the disorder, those prone to have the disorder, and those in whom the disorder is to be prevented. In some instances, a subject is successfully "treated" for a tumour/cancer according to the present invention if the subject shows one or more of the following: a reduction in the number of, or complete absence of, cancer cells; a reduction in the tumour size; inhibition of, or an absence of, cancer cell infiltration into peripheral organs including, for example, the spread of cancer into soft tissue and bone; inhibition of, or an absence of, tumour metastasis; inhibition of, or an absence of, tumour growth; reduced morbidity and mortality; reduction in tumourigenicity, tumourigenic frequency, or tumourigenic capacity of a tumour; reduction in the number or frequency of cancer stem cells in a tumour; differentiation of tumourigenic cells to a non-tumourigenic state; or some combination of effects.

As used herein, the term "subject" refers to any animal, including but not limited to humans, non-human primates, horses, canines, felines, rodents, and other vertebrates which are to be the recipient of a treatment for cancer. The terms "subject" and "patient" are used interchangeably herein.

As used herein, the term "tumour/cancer" refers to any mass of tissue that results from excessive cell growth, proliferation and/or survival, either benign (noncancerous) or malignant (cancerous), including pre-cancerous lesions.

The types of cancer treatable by the invention are not in any way limited, and include, for example, carcinoma, sarcoma, adenocarcinoma, melanoma, neural (blastoma, glioma), mesothelioma and reticuloendothelial, lymphatic or haematopoietic neoplastic disorders (e.g., myeloma, lymphoma or leukemia). In particular embodiments, the tumour can include a lung adenocarcinoma, lung carcinoma, diffuse or interstitial gastric carcinoma, colon adenocarcinoma, prostate adenocarcinoma, oesophagus carcinoma, breast carcinoma, pancreas adenocarcinoma, ovarian adenocarcinoma, adenocarcinoma of the adrenal gland, adenocarcinoma of the endometrium or uterine adenocarcinoma, but the type of cancer is preferably breast cancer.

In a preferred embodiment the cancer to be treated is selected from brain, breast, colon, lung, prostate and pancreatic cancer, and leukaemia. The cancer to be treated is preferably breast cancer, lung cancer or colon cancer. The cancer to be treated is most preferably breast cancer.

The definition of "breast cancer" is well known in the medical sciences. The skilled person will appreciate that breast cancer refers to any malignancy of the breast tissue of men or women, including, for example, carcinomas and sarcomas. Particular embodiments of breast cancer include ductal carcinoma in *situ* (DCIS), lobular carcinoma *in situ* (LCIS), or mucinous carcinoma. Breast cancer also refers to infiltrating ductal (IDC), lobular neoplasia or infiltrating lobular carcinoma (ILC).

Similarly, the definitions of "lung cancer" and "colon cancer" are well known in the medical sciences. The skilled person will appreciate that lung and colon cancers refer to any malignancy of the lung and colon tissues respectively of men or women, including, for example, carcinomas and sarcomas.

As used herein, the term "cancer cell" refers to a cell or immortalized cell line derived from tumour or cancer.

In one aspect of the invention is provided a method for determining the suitability of a subject, who has cancer and has already been treated with LDN, for treatment with a cannabinoid. The subject may have already been treated with a chemotherapeutic agent. In this aspect, a sample is to be obtained from the subject and contacted with a CB2-specific probe. The concentration of CB2 in the sample is thereby determined and compared with a reference measurement made before the first treatment phase.

As used herein, "suitability" refers to the property of having a high probability of treatment, as defined above, being successful, compared to those subjects deemed unsuitable by the test. A subset of subjects undergoing testing by the method envisaged in one aspect of this invention will be deemed to be unsuitable for the second phase of treatment. In no way is any part of the invention disallowed for use on such subjects. The method of determining suitability is intended to be considered by the skilled person alongside other tests known to them and exercising their intuition and judgement.

In certain embodiments, the biological "sample" obtained from the subject for use in the method is blood, plasma, serum, lymph fluid, a tissue, or cells derived from a tissue sample. Preferably however, the sample is a tumour biopsy obtained from the subject.

As used herein, the "probe" is any moiety which, on contacting the sample obtained from the subject, allows in some way the measurement of the concentration of CB2 in the sample. In one embodiment, this probe is an antibody or other CB2-binding molecule used in the provision of a purer solution of CB2 which can be analysed spectrophotometrically and through calibration using methods known to the skilled person, the concentration of CB2 in the original sample can be determined.

The invention will now be described with reference to the following non-limiting examples.

### Examples

### Example 1

One illustrative and non-limiting experiment was carried out on cultures of MCF7 (breast cancer) cells. In brief, either LDN at 10nM or 6BN at 1 µM or 10 µM was administered *in vitro* to a culture for two days, after which the expression levels of BAD, p21, opioid receptors kappa and mu, CB1, CB2, and GAPDH (as a loading control) were analysed via Western blot and subsequent quantification via density measurement of the bands.

In full, MCF7 cells were seeded into 6-well plates at a density of 1 x 105 /well and left to adhere over-night. Naltrexone (10 nM) or 6-β-naltrexol (1 µM or 10 µM) were added to the cells before harvesting cells for western blot analysis. Primary probing was performed with specific antibodies generated against BAD, p21, OPRK1, OPRK2, CBR-1 and CBR-2. BAD is Blc2-associate death promoter, a pro-apoptotic protein which, if upregulated in cancer cells, increases their tendency to be killed by a variety of therapies. Anti-GAPDH was used as a loading control. All antibodies were used at a dilution of 1:1000, followed by the appropriate HRP conjugated secondary antibodies also at a dilution of 1:1000. Bands were visualised by the SuperSignal chemiluminescent detection system, and densitometry of band intensity was determined using Adobe Photoshop CS3, v10.0, and normalised to the loading control.

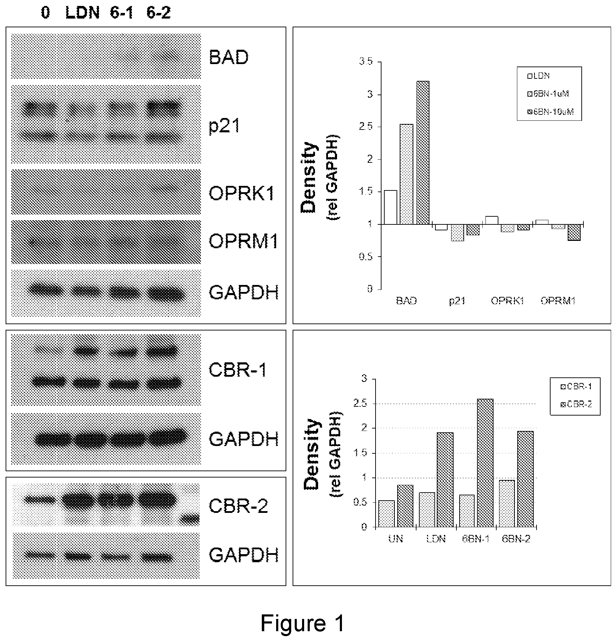

In response to all three treatments, levels of CB2 and BAD rose relative to the controls in which no drug was administered. For CB2, the rise was highest in response to administration of 10 µM 6BN. This can be seen in Figure 1.

### Example 2

Another illustrative and non-limiting experiment was carried out on cultures of MCF7 (breast cancer) cells. In brief, the cultures were treated with LDN or 6BN for two days, after which a cannabinoid was administered for a further two days. Cell number and viability were assessed on day four. The sequence of the administration for each therapeutic agent was reversed and the experiment repeated.

In full, MCF7 cells were seeded into 6-well plates at a density of 1.5 x 104 /well and left to adhere. Cells were then cultured with naltrexone (10 nM), 6-β-naltrexol (10 µM) or cannabidiol (10 µM). Drug-containing media was removed after 48 h, and cells were rinsed gently with drug-free medium. Fresh culture medium was then added to the cells supplemented with either naltrexone (10 nM), 6-β-naltrexol (10 µM) or cannabidiol (10 µM), as indicated in the graph. Cell number and viability were assessed a further 48 h later, with percentages of live and dead cells being discriminated by trypan blue dye exclusion. Data were then consolidated to compare the effect of sequence on overall effect.

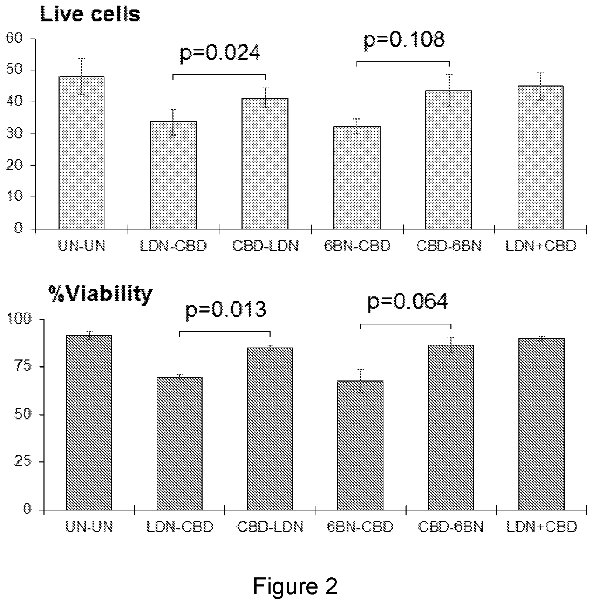

As can be seen in Figure 2, it was found that schedules in which LDN or 6BN were utilised before the cannabinoid had a greater effect on inhibiting the proliferation of the cancer.

### Example 3

• A549 (lung cancer) and HCT116 (colon cancer) cells were cultured with drugs according to the schedules in Table 1. The schedules lasted a total of 96h.
• Treatments were separated into two blocks, each lasting 48h. Due to the complications of cell testing using three different drugs in three different treatment phases, the CBD and LDN were administered together in one treatment phase and the chemotherapeutic agent in the other treatment phase.
• There was a washout period between the two blocks, where exhausted media containing drugs were removed, cells then washed, before fresh media was added that contained the next block of treatment.
• The chemotherapeutic agents were gemcitabine (GEM) or oxaliplatin (OXP), and used at an ~IC20 concentration (1uM). Low dose naltrexone (LDN) was used at 10nM and cannabinoid (CBD) was used at 1uM. "0" means nothing was administered and is used as a control.
• Cell number and percentage cell viability were assessed at 96h. Data were then consolidated to compare the effect of sequence on overall effect.

**Table 1: showing the schedules tested.**

| **Block 1 (0-48h)** | **Block 2 (48-96h)** |
|---|---|
| 0 | 0 |
| LDN+CBD | LDN+CBD |
| 0 | GEM |
| LDN+CBD | 0 |
| LDN+CBD | GEM |
| GEM | 0 |
| 0 | LDN+CBD |
| GEM | LDN+CBD |
| GEM | GEM |
| 0 | OXP (P) |
| LDN+CBD | OXP (P) |
| OXP (P) | 0 |
| OXP (P) | LDN+CBD |
| OXP (P) | OXP (P) |

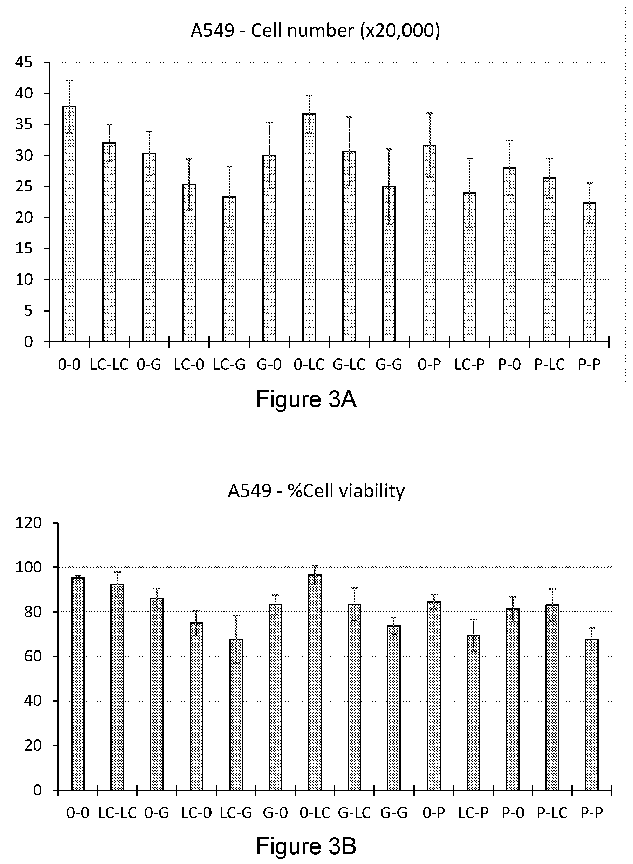

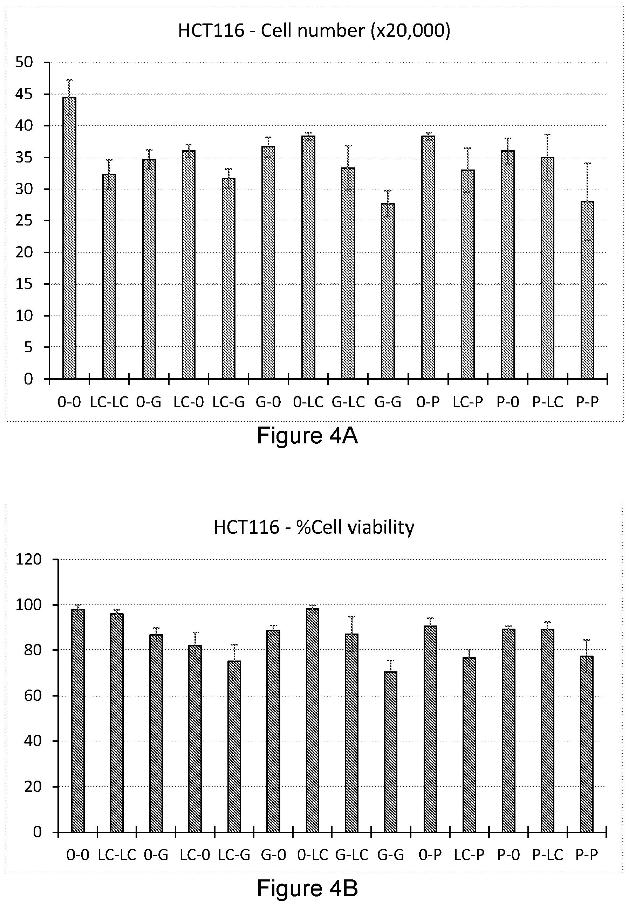

As shown in Figures 3 and 4 the use of CBD+LDN prior to chemotherapy is effective. Using LDN+CBD followed by a chemotherapeutic agent results in an improved or a similar effect to using only chemotherapy continuously over the 96 hour period (which thus uses a higher total amount of chemotherapeutic agent than the combination treatment). These results were observed for both cell types (A549 and HCT116) and both chemotherapeutic agents (GEM and OXP) tested. Treating cells with LDN+CBD before chemo resulted in lower numbers of cells and a decrease in %cell viability compared to cells that received no pre-treatment.

Thus, Example 3 shows that pre-treatment resulted in an effect that was similar to chemo used for 96h. This allows for an effective treatment regimen that maintains a better or comparable overall efficacy as continuous chemotherapy but uses a significantly reduced amount of chemotherapy, which is beneficial as it can then reduce the chemo-related toxicities that patients suffer.

### References

Cridge, B. & Rosengren, R (2013) Critical appraisal of the potential use of cannabinoids in cancer management. Cancer Management and Research 2013:5 301-313

## Claims

1. A pharmaceutical composition comprising:
(a) naltrexone, or
(b) a metabolite thereof selected from the group consisting of 6-β-naltrexol, 2-hydroxy-3-methoxy-6β-naltrexol and 2-hydroxy-3-methoxy-naltrexone, or
(c) an analogue selected from the group consisting of methylnaltrexone, naloxone, nalmefene and nalorphine,
for use in the treatment of cancer within a subject, wherein the subject is to be administered a chemotherapeutic agent before, during or following the treatment and wherein a therapeutically effective amount of the naltrexone or metabolite thereof or analogue of either is to be administered to the subject in a first treatment phase, and wherein after the first treatment phase the subject is to be administered a therapeutically effective amount of a cannabinoid in a second treatment phase.

2. A pharmaceutical composition comprising a cannabinoid for use in the treatment of cancer within a subject, wherein said subject is **characterised in** having undergone a first treatment phase during which the subject has been administered a therapeutically effective amount of:
(a) naltrexone, or
(b) a metabolite thereof selected from the group consisting of 6-β-naltrexol, 2-hydroxy-3-methoxy-6β-naltrexol and 2-hydroxy-3-methoxy-naltrexone, or
(c) an analogue selected from the group consisting of methylnaltrexone, naloxone, nalmefene and nalorphine,
and wherein following the first treatment phase a therapeutically amount of said cannabinoid is to be administered to the subject, and wherein the subject is to be administered a chemotherapeutic agent before, during or following the treatment.

3. A preparation comprising:
(a) naltrexone, or
(b) a metabolite thereof selected from the group consisting of 6-β-naltrexol, 2-hydroxy-3-methoxy-6β-naltrexol and 2-hydroxy-3-methoxy-naltrexone, or
(c) an analogue selected from the group consisting of methylnaltrexone, naloxone, nalmefene and nalorphine,
and a cannabinoid and a chemotherapeutic agent for use in the treatment of cancer within a subject, wherein the naltrexone or metabolite thereof or analogue of either is provided in a therapeutically effective amount to be administered in a first treatment phase, wherein the cannabinoid is provided in a therapeutically effective amount to be administered in a second treatment phase following the first treatment phase, and wherein the subject is to be administered a chemotherapeutic agent before, during or following the treatment.

4. The pharmaceutical composition for use according to any preceding claim, wherein the first treatment phase is for administration for at least two days.

5. The pharmaceutical composition or preparation for use according to any preceding claim, wherein the first treatment phase and second treatment phase are separated by a recovery phase, said recovery phase **characterised by** the absence of administration of the naltrexone or metabolite or analogue, the cannabinoid and the chemotherapeutic agent.

6. The pharmaceutical composition or preparation for use according to claim 5, wherein the recovery phase is for at least one day, preferably in the range of 1 to 7 days.

7. The pharmaceutical composition or preparation for use according to any preceding claim, wherein the second treatment phase is for administration for at least one day.

8. The pharmaceutical composition or preparation for use according to any preceding claim, wherein the composition or preparation comprises naltrexone or wherein the composition or preparation comprises 6-β-naltrexol.

9. The pharmaceutical composition or preparation for use according to any preceding claim, wherein the cannabinoid is selected from the list consisting of cannabidiol, cannabidiolic acid, cannabinol, cannabigerol, cannabivarin, tetrahydrocannabivarin, cannabidivarin, cannabichromene, arachidonoylethanolamine, 2-arachidonoylglycerol, 2-arachidonoyl glyceryl ether, *N*-arachidonoyl dopamine, virodhamine, dronabinol, nabilone, rimonabant or combinations thereof, preferably wherein the cannabinoid is cannabidiol.

10. The pharmaceutical composition or preparation for use according to any preceding claim, wherein the chemotherapeutic agent is selected from the group consisting of PI3-kinase inhibitors, AKT inhibitors, taxanes, antimetabolites, alkylating agents, cell cycle inhibitors, topoisomerase inhibitors and cytotoxic antibodies.

11. The pharmaceutical composition or preparation for use according to any preceding claim, wherein the chemotherapeutic agent is to be administered during the first treatment phase, or during the second treatment phase, or following the second treatment phase.

12. The pharmaceutical composition or preparation for use according to any preceding claim, wherein the cancer is breast cancer, lung cancer or colon cancer.

13. A method for determining the suitability of a subject with cancer for treatment with a cannabinoid in a second treatment phase, said subject **characterised in** having undergone a first treatment phase as defined in any preceding claim, the method comprising the steps of:
i. contacting a sample obtained from the subject after or during a recovery phase with a probe specific for CB2;
ii. determining the concentration of CB2 within the sample; and
iii. comparing the concentration of CB2 within the sample with a concentration of CB2 determined from a sample obtained from the subject before the first treatment phase,
wherein if the CB2 concentration has increased by at least two-fold after first treatment phase, the subject is suitable for the second treatment phase, and wherein the subject is or has been administered a chemotherapeutic agent.

14. The method of claim 13, wherein the sample is a tumour biopsy obtained from the patient.

15. The method according to either of claims 13 or 14, wherein the subject has breast cancer, lung cancer or colon cancer.

## Patentansprüche

1. Eine pharmazeutische Zusammensetzung, die Folgendes beinhaltet:
(a) Naltrexon oder
(b) einen Metaboliten davon, ausgewählt aus der Gruppe, bestehend aus 6-β-Naltrexol, 2-Hydroxy-3-methoxy-6β-naltrexol und 2-Hydroxy-3-methoxynaltrexon, oder
(c) ein Analogon, ausgewählt aus der Gruppe, bestehend aus Methylnaltrexon, Naloxon, Nalmefen und Nalorphin,
zur Verwendung bei der Behandlung von Krebs bei einem Individuum, wobei dem Individuum vor, während oder nach der Behandlung ein Chemotherapeutikum verabreicht werden soll und wobei dem Individuum in einer ersten Behandlungsphase eine therapeutisch wirksame Menge von dem Naltrexon oder Metaboliten davon oder Analogon von einem der beiden verabreicht werden soll und wobei dem Individuum nach der ersten Behandlungsphase eine therapeutisch wirksame Menge eines Cannabinoids in einer zweiten Behandlungsphase verabreicht werden soll.

2. Eine pharmazeutische Zusammensetzung, die ein Cannabinoid beinhaltet, zur Verwendung bei der Behandlung von Krebs bei einem Individuum, wobei das Individuum **dadurch gekennzeichnet ist, dass** es eine erste Behandlungsphase durchlaufen hat, in deren Verlauf dem Individuum eine therapeutisch wirksame Menge von Folgendem verabreicht wurde:
(a) Naltrexon oder
(b) einem Metaboliten davon, ausgewählt aus der Gruppe, bestehend aus 6-β-Naltrexol, 2-Hydroxy-3-methoxy-6β-naltrexol und 2-hydroxy-3-methoxynaltrexon, oder
(c) einem Analogon, ausgewählt aus der Gruppe, bestehend aus Methylnaltrexon, Naloxon, Nalmefen und Nalorphin,
und wobei dem Individuum im Anschluss an die erste Behandlungsphase eine therapeutische Menge des Cannabinoids verabreicht werden soll und wobei dem Individuum vor, während oder nach der Behandlung ein Chemotherapeutikum verabreicht werden soll.

3. Eine Zubereitung, die Folgendes beinhaltet:
(a) Naltrexon oder
(b) einen Metaboliten davon, ausgewählt aus der Gruppe, bestehend aus 6-β-Naltrexol, 2-Hydroxy-3-methoxy-6β-naltrexol und 2-Hydroxy-3-methoxynaltrexon, oder
(c) ein Analogon, ausgewählt aus der Gruppe, bestehend aus Methylnaltrexon, Naloxon, Nalmefen und Nalorphin,
und ein Cannabinoid und ein Chemotherapeutikum zur Verwendung bei der Behandlung von Krebs bei einem Individuum, wobei das Naltrexon oder der Metabolit davon oder das Analogon von einem der beiden in einer therapeutisch wirksamen Menge bereitgestellt wird, die in einer ersten Behandlungsphase verabreicht werden soll, wobei das Cannabinoid in einer therapeutisch wirksamen Menge bereitgestellt wird, die in einer zweiten Behandlungsphase im Anschluss an die erste Behandlungsphase verabreicht werden soll, und wobei dem Individuum vor, während oder nach der Behandlung ein Chemotherapeutikum verabreicht werden soll.

4. Die pharmazeutische Zusammensetzung zur Verwendung gemäß einem der vorhergehenden Ansprüche, wobei die erste Behandlungsphase für eine mindestens zwei Tage lange Verabreichung ist.

5. Die pharmazeutische Zusammensetzung oder Zubereitung zur Verwendung gemäß einem der vorhergehenden Ansprüche, wobei die erste Behandlungsphase und zweite Behandlungsphase durch eine Erholungsphase getrennt sind, wobei die Erholungsphase durch ein Fehlen der Verabreichung des Naltrexons oder des Metaboliten oder Analogons, des Cannabinoids und des Chemotherapeutikums gekennzeichnet ist.

6. Die pharmazeutische Zusammensetzung oder Zubereitung zur Verwendung gemäß Anspruch 5, wobei die Erholungsphase für mindestens einen Tag, bevorzugt im Bereich von 1 bis 7 Tagen, ist.

7. Die pharmazeutische Zusammensetzung oder Zubereitung zur Verwendung gemäß einem der vorhergehenden Ansprüche, wobei die zweite Behandlungsphase für eine mindestens einen Tag lange Verabreichung ist.

8. Die pharmazeutische Zusammensetzung oder Zubereitung zur Verwendung gemäß einem der vorhergehenden Ansprüche, wobei die Zusammensetzung oder Zubereitung Naltrexon beinhaltet oder wobei die Zusammensetzung oder Zubereitung 6-β-Naltrexol beinhaltet.

9. Die pharmazeutische Zusammensetzung oder Zubereitung zur Verwendung gemäß einem der vorhergehenden Ansprüche, wobei das Cannabinoid aus der Liste ausgewählt ist, die aus Cannabidiol, Cannabidiolsäure, Cannabinol, Cannabigerol, Cannabivarin, Tetrahydrocannabivarin, Cannabidivarin, Cannabichromen, Arachidonoylethanolamin, 2-Arachidonoylglycerol, 2-Arachidonoylglycerylether, *N-*Arachidonoyldopamin, Virodhamin, Dronabinol, Nabilon, Rimonabant oder Kombinationen davon besteht, wobei das Cannabinoid bevorzugt Cannabidiol ist.

10. Die pharmazeutische Zusammensetzung oder Zubereitung zur Verwendung gemäß einem der vorhergehenden Ansprüche, wobei das Chemotherapeutikum aus der Gruppe ausgewählt ist, die aus P13-Kinasehemmern, AKT-Hemmern, Taxanen, Antimetaboliten, alkylierenden Mitteln, Zellzyklushemmern, Topoisomerasehemmern und zytotoxischen Antikörpern besteht.

11. Die pharmazeutische Zusammensetzung oder Zubereitung zur Verwendung gemäß einem der vorhergehenden Ansprüche, wobei das Chemotherapeutikum während der ersten Behandlungsphase oder während der zweiten Behandlungsphase oder im Anschluss an die zweite Behandlungsphase verabreicht werden soll.

12. Die pharmazeutische Zusammensetzung oder Zubereitung zur Verwendung gemäß einem der vorhergehenden Ansprüche, wobei der Krebs Brustkrebs, Lungenkrebs oder Kolonkrebs ist.

13. Ein Verfahren zur Bestimmung der Eignung eines Individuums mit Krebs für die Behandlung mit einem Cannabinoid in einer zweiten Behandlungsphase, wobei das Individuum **dadurch gekennzeichnet ist, dass** es eine erste Behandlungsphase wie in einem der vorherigen Ansprüche definiert durchlaufen hat, wobei das Verfahren die folgenden Schritte beinhaltet:
i. In-Kontakt-Bringen einer Probe, die von dem Individuum nach oder während einer Erholungsphase erhalten wurde, mit einer für CB2 spezifischen Sonde;
ii. Bestimmen der Konzentration von CB2 innerhalb der Probe; und
iii. Vergleichen der Konzentration von CB2 innerhalb der Probe mit einer Konzentration von CB2, die aus einer vor der ersten Behandlungsphase von dem Individuum erhaltenen Probe bestimmt wurde,
wobei das Individuum, wenn die CB2-Konzentration nach der ersten Behandlungsphase um mindestens das Zweifache angestiegen ist, für die zweite Behandlungsphase geeignet ist, und wobei dem Individuum ein Chemotherapeutikum verabreicht wird oder verabreicht worden ist.

14. Das Verfahren gemäß Anspruch 13, wobei die Probe eine von dem Patienten erhaltene Tumorbiopsie ist.

15. Das Verfahren gemäß einem der Ansprüche 13 oder 14, wobei das Individuum Brustkrebs, Lungenkrebs oder Kolonkrebs hat.

## Revendications

1. Composition pharmaceutique comprenant :
(a) de la naltrexone, ou
(b) un métabolite de celle-ci sélectionné parmi le groupe composé de 6-β-naltrexol, 2-hydroxy-3-méthoxy-6β-naltrexol et 2-hydroxy-3-méthoxy-naltrexone, ou
(c) un analogue sélectionné parmi le groupe composé de méthylnaltrexone, naloxone, nalméfène et nalorphine,
pour utilisation dans le traitement d'un cancer chez un sujet, où le sujet doit recevoir un agent chimiothérapeutique avant, pendant ou après le traitement et où une quantité thérapeutiquement efficace de la naltrexone ou d'un métabolite de celle-ci ou d'un analogue de l'un ou l'autre doit être administrée au sujet dans une première phase de traitement, et où après la première phase de traitement le sujet doit recevoir une quantité thérapeutiquement efficace d'un cannabinoïde dans une deuxième phase de traitement.

2. Composition pharmaceutique comprenant un cannabinoïde pour utilisation dans le traitement d'un cancer chez un sujet, où ledit sujet est caractérisé comme ayant suivi une première phase de traitement durant laquelle le sujet a reçu une quantité thérapeutiquement efficace :
(a) de naltrexone, ou
(b) d'un métabolite de celle-ci sélectionné parmi le groupe composé de 6-β-naltrexol, 2-hydroxy-3-méthoxy-6β-naltrexol et 2-hydroxy-3-méthoxy-naltrexone, ou
(c) d'un analogue sélectionné parmi le groupe composé de méthylnaltrexone, naloxone, nalméfène et nalorphine,
et où suite à la première phase de traitement, une quantité thérapeutiquement efficace dudit cannabinoïde doit être administrée au sujet, et où le sujet doit recevoir un agent chimiothérapeutique avant, pendant ou après le traitement.

3. Préparation comprenant :
(a) de la naltrexone, ou
(b) un métabolite de celle-ci sélectionné parmi le groupe composé de 6-β-naltrexol, 2-hydroxy-3-méthoxy-6β-naltrexol et 2-hydroxy-3-méthoxy-naltrexone, ou
(c) un analogue sélectionné parmi le groupe composé de méthylnaltrexone, naloxone, nalméfène et nalorphine,
et un cannabinoïde et un agent chimiothérapeutique pour utilisation dans le traitement d'un cancer chez un sujet, où la naltrexone ou un métabolite de celle-ci ou un analogue de l'un ou de l'autre est fourni en une quantité thérapeutiquement efficace à administrer dans une première phase de traitement, où le cannabinoïde est fourni en une quantité thérapeutiquement efficace à administrer dans une deuxième phase de traitement suite à la première phase de traitement, et où le sujet doit recevoir un agent chimiothérapeutique avant, pendant ou après le traitement.

4. Composition pharmaceutique pour utilisation selon l'une quelconque des revendications précédentes, où la première phase de traitement doit être administrée pendant deux jours au moins.

5. Composition pharmaceutique ou préparation pour utilisation selon l'une quelconque des revendications précédentes, où la première phase de traitement et la deuxième phase de traitement sont séparées par une phase de récupération, ladite phase de récupération étant **caractérisée par** l'absence d'administration de la naltrexone ou du métabolite ou de l'analogue, du cannabinoïde et de l'agent chimiothérapeutique.

6. Composition pharmaceutique ou préparation pour utilisation selon la revendication 5, où la phase de récupération est d'un jour au moins, de préférence dans la page de 1 à 7 jours.

7. Composition pharmaceutique ou préparation pour utilisation selon l'une quelconque des revendications précédentes, où la deuxième phase de traitement est pour administration pendant 1 jour au moins.

8. Composition pharmaceutique ou préparation pour utilisation selon l'une quelconque des revendications précédentes, où la composition ou préparation comprend de la naltrexone ou bien où la composition ou préparation comprend du 6-β-naltrexol.

9. Composition pharmaceutique ou préparation pour utilisation selon l'une quelconque des revendications précédentes, où le cannabinoïde est sélectionné parmi la liste composée de cannabidiol, acide cannabidiolique, cannabinol, cannabigérol, cannabivarine, tétrahydrocannabivarine, cannabidivarine, cannabichromène, arachidonoyléthanolamine, 2-arachidonoylglycérol, 2-arachidonoyl glycéryl éther, *N-*arachidonoyl dopamine, virodhamine, dronabinol, nabilone, rimonabant ou des combinaisons de ceux-ci, de préférence le cannabinoïde est du cannabidiol.

10. Composition pharmaceutique ou préparation pour utilisation selon l'une quelconque des revendications précédentes, où l'agent chimiothérapeutique est sélectionné parmi le groupe composé d'inhibiteurs de PK3-kinase, inhibiteurs d'AKT, taxanes, antimétabolites, agents alkylants, inhibiteurs du cycle cellulaire, inhibiteurs de la topoisomérase et anticorps cytotoxiques.

11. Composition pharmaceutique ou préparation pour utilisation selon l'une quelconque des revendications précédentes, où l'agent chimiothérapeutique doit être administré pendant la première phase de traitement ou pendant la deuxième phase de traitement, ou suite à la deuxième phase de traitement.

12. Composition pharmaceutique ou préparation pour utilisation selon l'une quelconque des revendications précédentes, où le cancer est un cancer du sein, un cancer du poumon ou un cancer du côlon.

13. Procédé de détermination de l'aptitude d'un sujet atteint d'un cancer à un traitement avec un cannabinoïde dans une deuxième phase de traitement, ledit sujet étant caractérisé comme ayant suivi une première phase de traitement telle que définie selon l'une quelconque des revendications précédentes, le procédé comprenant les étapes consistant à :
i. mettre un échantillon obtenu du sujet après ou pendant une phase de récupération en contact avec une sonde spécifique pour le CB2 ;
ii. déterminer la concentration de CB2 dans l'échantillon ; et
iii. comparer la concentration de CB2 dans l'échantillon à une concentration de CB2 déterminée d'un échantillon obtenu du sujet avant la première phase de traitement,
dans lequel, si la concentration de CB2 a augmenté d'au moins deux fois après la première phase de traitement, le sujet est apte à la deuxième phase de traitement, et dans lequel le sujet reçoit ou a reçu un agent chimiothérapeutique.

14. Procédé selon la revendication 13, dans lequel l'échantillon est une biopsie de tumeur obtenue du patient.

15. Procédé selon la revendication 13 ou la revendication 14, où le sujet a un cancer du sein, un cancer du poumon ou un cancer du côlon.
